(19) **Europäisches Patentamt European Patent Office Office européen des brevets**

(11) **EP 1 621 220 B1**

(12) **EUROPEAN PATENT SPECIFICATION**

(45) Date of publication and mention of the grant of the patent:
**19.11.2014 Bulletin 2014/47**

(21) Application number: **04729289.1**

(22) Date of filing: **23.04.2004**

(51) Int Cl.:
***A61M 1/36*** (2006.01)

(86) International application number:
**PCT/JP2004/005953**

(87) International publication number:
**WO 2004/098680 (18.11.2004 Gazette 2004/47)**

(54) **LOW DENSITY LIPOPROTEIN/FIBRINOGEN ADSORBENT AND ADSORPTION APPARATUS CAPABLE OF WHOLE BLOOD TREATMENT**

LIPOPROTEIN NIEDERER DICHTE/FIBRINOGEN ADSORBENS UND GERÄT FÜR DIE VOLLBLUTBEHANDLUNG

ADSORBANT DE LIPOPROTEINE/FIBRINOGENE DE FAIBLE DENSITE ET APPAREIL D'ADSORPTION PERMETTANT UN TRAITEMENT DU SANG TOTAL

(84) Designated Contracting States:
**AT BE BG CH CY CZ DE DK EE ES FI FR GB GR HU IE IT LI LU MC NL PL PT RO SE SI SK TR**

(30) Priority: **08.05.2003 JP 2003130641**

(43) Date of publication of application:
**01.02.2006 Bulletin 2006/05**

(73) Proprietor: **KANEKA CORPORATION Osaka (JP)**

(72) Inventors:
• **NAKATANI, Masaru**
  **Settsu-shi,**
  **Osaka 5660072 (JP)**
• **KOBAYASHI, Akira**
  **Settsu-shi,**
  **Osaka 5660072 (JP)**
• **NISHIMOTO, Takehiro**
  **Settsu-shi,**
  **Osaka 5660072 (JP)**
• **FURUYOSHI, Shigeo**
  **Settsu-shi,**
  **Osaka 5660072 (JP)**

(74) Representative: **Hoffmann Eitle Patent- und Rechtsanwälte PartmbB Arabellastraße 30 81925 München (DE)**

(56) References cited:
| | |
|---|---|
| EP-A1- 0 056 977 | EP-A2- 0 225 867 |
| WO-A1-91/01808 | JP-A- 5 269 203 |
| JP-A- 6 296 859 | JP-A- 7 136 256 |
| JP-A- 60 090 039 | JP-A- 63 115 572 |
| JP-A- 2001 316 420 | US-A1- 2002 164 644 |

**Description**

Technical Field

The present invention relates to an adsorbent for adsorbing low-density lipoproteins and fibrinogen present in a body fluid to decrease the concentrations thereof in the body fluid. Furthermore, the present invention relates to an adsorber using the adsorbent for low-density lipoproteins and fibrinogen in a body fluid. Particularly, the present invention relates to an adsorbent capable of whole blood treatment.

Background Art

In recent years, patients affected by arteriosclerosis have increased in number with westernization of eating habits and aging. It is well known that low-density lipoproteins (LDL) and very low-density lipoproteins (VLDL) are rich in cholesterol and thus cause arteriosclerosis. It is also the fact that arteriosclerosis highly develops in patients with hyperlipemia or hypercholesterolemia. On the other hand, high-density lipoproteins (HDL) are known as a retardation factor against arteriosclerosis.

Although therapies for these diseases include a dietary therapy and a drug therapy, a therapy applied to a patient who cannot be effectively treated by these therapies comprises extracorporeally removing low-density lipoproteins from the blood by adsorption. In particular, a therapy of perfusing the blood plasma separated from the blood through an adsorber filled with an adsorbent comprising cellulose beads with immobilized dextran sulfate to remove the low-density lipoproteins is widely used with a high curative effect.

On the other hand, it has been reported that a fibrinogen concentration is correlated with the incidence of coronary artery diseases and cerebral apoplexy (W. B. Kannel et al., The Journal of the American Medical Association, Vol. 258, pp. 1183-1186, 1987). In order to prevent the occurrence of these diseases related to arteriosclerosis, it is desired to decrease the fibrinogen concentration as well as the concentration of low-density lipoproteins.

In particular, arteriosclerosis causing the occlusion of a peripheral blood vessel is referred to as "arteriosclerosis obliterans". In this disease, the peripheral blood vessel is narrowed or occluded to worsen the circulation of peripheral blood, thereby causing symptoms such as coldness in the limbs, numbness, intermittent claudication, a pain at rest, an ulcer, mortification, and the like, leading to limb amputation. It has been also reported that a patient with the arteriosclerosis obliterans having such lesions in the peripheral blood vessel has a higher fibrinogen concentration than that of a healthy adult (P. Poredos et al., Angiology, Vol. 47, No. 3, pp. 253-259, 1996). In treatment of the arteriosclerosis obliterans, therefore, it is also desired to decrease the fibrinogen concentration as well as the concentration of low-density lipoproteins.

As described above, a therapy desired for a patient with arteriosclerosis, particularly arteriosclerosis obliterans, comprises decreasing the concentrations of low-density lipoproteins and fibrinogen in blood. The above-described therapy of removing the low-density lipoproteins from blood plasma by adsorption on the adsorbent comprising cellulose beads with immobilized dextran sulfate is excellent in adsorption of the low-density lipoproteins, but the therapy is not necessarily sufficient for decreasing the fibrinogen concentration. In some cases, double filtration plasmapheresis is applied. In this method, the plasma separated by a plasma separator is introduced into a plasma filter membrane to remove an unfiltered substance, i.e., a substance larger than the pore diameter of the membrane, together with water. This method can securely remove low-density lipoproteins and fibrinogen, but it is disadvantageous in that the filtration system used requires electrolytic transfusion (fluid replacement), and even if a complicated operation such as temperature control, recirculation, or the like is performed, selectivity for a substance to be removed is lower than that of adsorption, thereby removing useful substances other than low-density lipoproteins and fibrinogen, for example, albumin, immunoglobulin such as IgG, HDL-cholesterol, and the like (Yoshie Konno, et al., Japanese Journal of Apheresis, Vo. 22, No. 1, pp. 44-50, 2003). Furthermore, it has been reported that a therapy referred to as a "heparin precipitation method" has been developed for removing low-density lipoproteins and fibrinogen. However, this method comprises a complicated operation and is not popularized as a general therapy.

Also, it is known that fibrinogen and low-density lipoproteins can be removed with an adsorbent comprising a cross-linked porous material containing a compound in its surfaces, the compound having a hydrophobic structure and an anionic functional group (Japanese Unexamined Patent Application Publication No. 7-136256). Although the adsorbent has excellent adsorption ability for fibrinogen, the adsorption ability for low-density lipoproteins is not sufficient. In order to exhibit the clinically sufficient adsorption ability of the adsorbent, a large amount of the adsorbent must be used. Therefore, the amount of the blood taken out from a body in a therapy is increased to increase the probability of occurrence of a blood pressure drop in the therapy. This document also discloses a preferred method for using the adsorbent in which plasma is separated from blood by a plasma separator and then treated from the viewpoint of influences on blood cell components such as platelets.

Therefore, the conventional methods for decreasing the low-density lipoproteins and fibrinogen are disadvantageous in that the methods comprise complicated operations due to a plasma separation system and have low performance, and useful substances are also removed. On the other hand, a system for direct whole blood treatment without plasma separation from blood has recently attracted attention as an extracorporeal circulation therapy using an adsorbent in view of simplicity of operations and shortening of the therapy time. The direct whole blood treatment system does not require plasma separation using a plasma separator or the like, and is capable of direct treatment of the blood anticoagulated with an anticoagulant. Therefore, the circuit is very simple, and the target substance can be effectively adsorbed within a short time. Consequently, a decrease in burden to a patient and medical staff is expected.

However, the direct whole blood treatment system is required to decrease the interaction between the adsorbent and blood cells and decrease the influence on the blood cell components as much as possible. In the direct whole blood treatment, it is most important to inhibit the activation of leukocytes and platelets as much as possible. When the activation is low, a loss of these blood cells can be prevented. Particularly, when a blood vessel is damaged, the platelets adhere to the damaged site, and a fibrinogen receptor is expressed on the surface to form thrombus due to cross-linking of the platelets with fibrinogen. The thrombus possibly covers the damaged site to prevent a blood leakage. Therefore, the technique for adsorbing fibrinogen by whole blood treatment is considered very difficult. With respect to the above-described adsorbent comprising a cross-linked porous material containing a compound in its surfaces, the compound having a hydrophobic structure and an anionic functional group, there is no concrete study of a method of direct whole blood treatment, and a plasma treatment system is considered preferable (Japanese Unexamined Patent Application Publication No. 7-136256).

European patent application EP 0 225 867 discloses an adsorbent for removal of low density lipoproteins from body fluid, the absorbent comprising a polyanion compound which is immobilized on a water-insoluble porous polymer.

United States patent application US 2002/0164644 discloses an adsorbent of high-mobility-group proteins for removing these proteins from body fluid. The adsorbent comprises a water-insoluble carrier on which at least one substance having a hydrogen-bondable functional group and/or a hydrophobic functional group is immobilized.

As described above, there has been no conventional method for effectively removing low-density lipoproteins and fibrinogen by a very simple operation of whole blood treatment without plasma separation and a loss of other useful substances. Therefore, the development of such a method has been demanded.

Disclosure of the Invention

In order to solve the above problems, the present invention provides an adsorbent for efficiently adsorbing low-density lipoproteins and fibrinogen from a body fluid, particularly whole blood, to decrease the concentrations of the low-density lipoproteins and fibrinogen in the body fluid while minimizing a loss of useful substances such as albumin and HDL. The present invention further provides an adsorber comprising the adsorbent for adsorbing low-density lipoproteins and fibrinogen in a body fluid. Particularly, the present invention provides an adsorbent and adsorber capable of minimizing a loss of blood cells and safely treating whole blood.

The inventors carried out intensive research of an adsorbent capable of minimizing a loss of useful substances such as albumin and HDL and effectively adsorbing low-density lipoproteins and fibrinogen by whole blood treatment. As a result, the inventors found an adsorbent comprising a tryptophan derivative and a polyanionic compound which are immobilized on a water-insoluble porous carrier, wherein a predetermined amount of the polyanionic compound is immobilized, and the molar ratio of the amount of the immobilized tryptophan to the amount of the immobilized polyanionic compound is in a specified range. Also, inventors found that the adsorbent is capable of safe whole blood treatment for effectively adsorbing low-density lipoproteins and fibrinogen in a body fluid while minimizing a loss of blood cells. This finding resulted in the achievement of the present invention.

In a first aspect of the present invention, an adsorbent capable of whole blood treatment for adsorbing low-density lipoproteins and fibrinogen comprises a tryptophan derivative and a polyanionic compound which are immobilized on a water-insoluble porous carrier, wherein the amount of the immobilized polyanionic compound is 0.10 $\mu$mol to 1.5 $\mu$mol per milliliter of wet volume of the adsorbent, and the molar ratio of the amount of the immobilized tryptophan derivative to the amount of the immobilized polyanionic compound per milliliter of wet volume of the adsorbent is 1 to 70. In a second aspect of the present invention, an adsorber capable of whole blood treatment for absorbing low-density lipoproteins and fibrinogen comprises a container having a fluid inlet and outlet and a means for preventing an outflow of the adsorbent to the outside, the container being filled with the adsorbent for low-density lipoproteins and fibrinogen.

In the present invention, the term "body fluid" means blood or plasma.

In the present invention, the term "polyanionic compound" means a compound having a plurality of anionic functional groups in its molecule. In the present invention, examples of the anionic functional groups include functional groups negatively charged at neutral pH, such as a carboxyl group, a sulfonate group, a sulfate group, and a phosphate group. Among these functional groups, from the viewpoint of adsorption ability, a carboxyl group, a sulfonate group, and a sulfate group are preferred. In view of highest adsorption ability, a sulfate group is particularly preferred.

Representative examples of the polyanionic compound include synthetic polyanionic compounds such as polyacrylic acid, polyvinylsulfonic acid, polystyrenesulfonic acid, polyglutamic acid, polyasparaginic acid, polymethacrylic acid, polyphosphoric acid, and styrene-maleic acid copolymers; synthetic acid polysaccharides such as dextran sulfate and carboxymethyl cellulose; acid tissue-derived acid mucopolysaccharides having sulfate groups, such as chondroitin sulfate, dermantan sulfate, and keratan sulfate; acid mucopolysaccharides having N-sulfonate groups or sulfate groups, such as heparin and heparan sulfate; tissue-derived polysaccharides having anionic functional groups, such as chondroitin and phosphomannan; and tissue-derived nucleic acids such as deoxyribonucleic acid and ribonucleic acid. However, the polyanionic compound is not limited to these representative examples.

Among these representative compounds, it is practical to use synthetic compounds rather than directly using tissue-derived compounds because a high-purity substance can be obtained at low cost, and the amount of the anionic functional groups introduced can be controlled. From these viewpoints, synthetic polyanionic compounds such as polyacrylic acid, polyvinylsulfuric acid, polyvinylsulfonic acid, polystyrenesulfonic acid, polyglutamic acid, polyasparaginic acid, polymethacrylic acid, polyphosphoric acid, and styrene-maleic acid copolymers; and synthetic acid polysaccharides such as dextran sulfate and carboxymethyl cellulose are preferably used. In particular, from the viewpoint of low cost, polyacrylic acid, polystyrenesulfonic acid, and dextran sulfate are more preferred, and dextran sulfate is most preferred from the viewpoint of safety.

The molecular weight of the polyanionic compound is preferably 1000 or more, and more preferably 3000 or more in view of affinity for the low-density lipoproteins and the fibrinogen adsorbing ability in combination with tryptophan. Although the upper limit of the molecular weight of the polyanionic compound is not particularly limited, the upper limit is preferably 1,000,000 or less from the practical viewpoint.

In the present invention, any one of various methods for immobilizing the polyanionic compound to the water-insoluble porous carrier may be used. Representative examples of the method include (1) a grafting method using radiation or electron beams for covalently bonding the polyanionic compound to the surfaces of the water-insoluble porous carrier, and (2) a chemical method of covalently bonding the polyanionic compound through the functional groups of the water-soluble porous carrier.

In the present invention, in view of the structure of the adsorbent in which the polyanionic compound and the tryptophan derivative are immobilized, the chemical method of covalently bonding the polyanionic compound through the functional groups is simpler and preferred because the tryptophan derivative can be immobilized by the same method.

In the present invention, examples of the tryptophan derivative include tryptophan, tryptophan esters such as tryptophan ethyl ester and tryptophan methyl ester, and compounds having indole rings and structures similar to tryptophan, such as tryptamine and tryptophanol. The tryptophan derivative may be an L-isomer, a D-isomer, a DL-isomer, or a mixture thereof. Alternatively, a mixture of at least two tryptophan derivatives may be used. Among these tryptophan derivatives, tryptophan is preferred from the viewpoint of safety, and L-tryptophan is most preferable in practical use because safety data is abundant, and tryptophan is a natural amino acid, most inexpensive, and readily available.

In the present invention, as the method for immobilizing the tryptophan derivative, the chemical method of covalently boding the tryptophan derivative through the functional groups of the water-insoluble porous carrier is preferably used.

In the present invention, the amount of the immobilized polyanionic compound must be 0.10 μmol to 1.5 μmol per milliliter of wet volume of the adsorbent, and the molar ratio of the amount of the immobilized tryptophan derivative to the amount of the immobilized polyanionic compound must be 1 to 70.

In the present invention, the molar ratio (TR/PA ratio) of the amount of the immobilized tryptophan derivative to the amount of the immobilized polyanionic compound is calculated according to the following equation:

$$\text{TR/PA ratio} = \text{molar number of the immobilized}$$

tryptophan derivative per milliliter of wet volume of the adsorbent/molar number of the immobilized polyanionic compound per milliliter of wet volume of the adsorbent.

The inventors carried out intensive study on the amount of the immobilized polyanionic compound, the amount of the immobilized tryptophan derivative, low-density lipoproteins and fibrinogen, and blood cell passing property in direct whole blood treatment. As a result, it was surprisingly found that when the amount of the immobilized polyanionic compound is controlled to 0.10 μmol to 1.5 μmol per milliliter of wet volume of the adsorbent, and the molar ratio TR/PA is controlled to 1 to 70, high adsorption ability is exhibited for the low-density lipoproteins and fibrinogen, and passing property to leukocytes and platelets is excellent.

In the present invention, the amount of the immobilized polyanionic compound is 0.10 μmol to 1.5 μmol per milliliter (wet volume) of the adsorbent. With the amount of less than 0.10 μmol, the passing property to leukocytes and platelets is low to decrease the number of the leukocytes in the pooled blood in whole blood perfusion. With the amount of over 1.5 μmol, the fibrinogen adsorbing ability is less exhibited even when the tryptophan derivative is immobilized.

In view of high blood cell passing property and high adsorption ability, the amount of the immobilized polyanionic compound is preferably 0.12 $\mu$mol to 1.0 $\mu$mol, and more preferably 0.15 $\mu$mol to 0.50 $\mu$mol.

In the present invention, the molar ratio (TR/PA ratio) of the amount of the immobilized tryptophan derivative to the amount of the immobilized polyanionic compound is 1 to 70. With the TR/PA ratio of less than 1, the fibrinogen adsorbing ability of the tryptophan derivative is less exhibited. Conversely, with the TR/PA ratio over 70, the passing property to leukocytes and platelets gradually worsens to decrease the number of the leukocytes in the pooled blood in whole blood perfusion. In view of high blood cell passing property and high adsorption ability, the molar ratio is preferably 5 to 60, and more preferably 10 to 50.

In the present invention, the wet volume of the adsorbent is determined as follows: The adsorbent is immersed in water and transferred as slurry into a measuring container such as a measuring cylinder, and the adsorbent slurry is spontaneously settled in the measuring container. Then, a rubber mat is placed for preventing breakage of the measuring container, and the measuring container is gently dropped about 5 to 10 times onto the mat from a height of about 5 to 10 cm in the vertical direction (so that the settled adsorbent does not extremely rise) to apply vibration to the adsorbent. After the measuring container is allowed to stand for 15 minutes or more, the volume of the adsorbent settled is measured. The operation of vibration and standing is repeated, and the volume of the adsorbent settled is measured as the wet volume when the volume of the adsorbent settled is not changed.

In the present invention, examples of the method for measuring the amount of the immobilized polyanionic compound include a method of determining the content of an element in the polyanionic compound in the adsorbent (for example, when dextran sulfate is the polyanionic compound, the sulfur content in the adsorbent is determined), and a method of measuring a decrease in amount of a pigment in a pigment solution in contact with the adsorbent, the pigment having the property of bonding to the polyanionic compound. Among these methods, the method using the pigment solution is capable of simply and precisely measuring the amount of the immobilized polyanionic compound. The method will be described in detail below in EXAMPLE 1. When the polyanionic compound is dextran sulfate or polyacrylic acid, the amount of the immobilized compound can be simply measured from the amount of the toluidine blue adsorbed on the adsorbent in contact with a toluidine blue solution because the compound has the property of bonding to the toluidine blue.

In the present invention, the amount of the immobilized tryptophan derivative can be determined by using the property that a color is generated when an aldehyde such as p-dimethylbenzaldehyde is condensed with the indole ring in the molecule of the tryptophan derivative under a strong acid condition (Amino Acid Fermentation (second volume) edited by Koichi Yamada, pp. 43-45, Kyoritsu Shupppan, 1972). The amount of the immobilized tryptophan derivative can also be determined by a method using the property that fluorescent light with a peak at about 350 nm is emitted when the indole ring in the molecule of the tryptophan derivative is excited with light at about 280 nm. When the carrier comprises a compound not containing nitrogen, the amount can be measured by determining the nitrogen content in the adsorbent, as will be descried in detail below in the method of EXAMPLE 1.

In the present invention, the water-insoluble porous carrier is water-insoluble at normal temperature and normal pressure, and has fine holes of an appropriate size, i.e., a porous structure. As the shape of the water-insoluble porous carrier, any one of a spherical shape, a granular shape, a flat membrane, a fibrous shape, a hollow fiber, and the like may be effectively used. However, a spherical shape or a granular shape is preferably used from the viewpoint of ease of handling.

When the water-insoluble porous carrier has a spherical shape or granular shape, the average particle size of the carrier is preferably as large as possible in view of the point that the adsorbent of the present invention is capable of whole blood treatment. However, in view of adsorption efficiency, the average particle size is preferably as small as possible. In the present invention, in order to permit the whole blood treatment and the exhibition of high adsorption efficiency, the average particle size of the adsorbent is preferably 100 $\mu$m to 1000 $\mu$m. Also, from the viewpoint that high blood cell passing property and adsorption efficiency can be exhibited, the average particle size of the adsorbent is more preferably 200 $\mu$m to 800 $\mu$m, and most preferably 400 $\mu$m to 600 $\mu$m.

The water-insoluble porous carrier preferably has a molecular weight exclusion limit of $5 \times 10^5$ or more for globular proteins. As described in a book (Size Exclusion Chromatography, written by Sadao Mori, Kyoritsu Shuppan), the molecule weight exclusion limit means the molecular weight of a molecule having the smallest molecular weight among the molecules not entering in fine pores (excluded) when a sample having various molecular weights is flowed in size exclusion chromatography. When the molecular weight exclusion limit for globular proteins is less than $5 \times 10^5$, it is not practical because of the low adsorption ability for fibrinogen and low-density lipoproteins. When the molecular weight exclusion limit for globular proteins is over $1 \times 10^8$, the pore size is excessively large to decrease the surface area contributing to adsorption, thereby decreasing the adsorption ability for fibrinogen and low-density lipoproteins. In the present invention, therefore, the molecular weight exclusion limit of the water-soluble porous carrier for globular proteins is preferably $5 \times 10^5$ to $1 \times 10^8$, and more preferably $1 \times 10^6$ to $1 \times 10^8$, -and most preferably $2 \times 10^6$ to $1 \times 10^8$ from the viewpoint of exhibition of adsorption ability.

In the present invention, the water-insoluble porous carrier preferably has functional groups usable for bonding for immobilizing the polyanionic compound and the tryptophan derivative. Representative examples of the functional groups include an amino group, an amide group, a carboxyl group, an acid anhydride group, a succinimide group, a hydroxyl group, a thiol group, an aldehyde group, a halogen group, an epoxy group, a silanol group, and a tresyl group. However, the functional groups are not limited to these groups. The water-insoluble porous carrier may be activated by a method, for example, a halogenation-cyanidation method, an epichlorohydrin method, a bisepoxide method, or a bromoacetyl bromide method. Among these methods, the epichlorohydrin method is most preferably used from the viewpoint of practical use and safety.

In the present invention, it is undesirable that the water-insoluble porous carrier is excessively soft or easily broken. When consolidation occurs during flowing of a body fluid, a sufficient flow rate of the body fluid cannot be obtained to extend the treatment time and fail to continue the treatment. Therefore, in order to prevent the consolidation of the adsorbent, the adsorbent preferably has sufficient mechanical strength (hardness). The term "hardness" means that when an aqueous liquid is flowed through a cylindrical column uniformly filled with the adsorbent, the pressure drop and the flow rate have a linear relationship up to at least $0.3 \ kgf/cm^2$, as shown below in a reference example.

In the present invention, the material of the water-insoluble porous carrier is not particularly limited. However, representative examples of the material include organic carriers comprising polysaccharides, such as cellulose, cellulose acetate, and dextrin; synthetic polymers such as polystyrene, styrene-divinylbenzene copolymers, polyacrylamide, polyacrylic acid, polymethacrylic acid, polyacrylic acid esters, polymethacrylic acid esters, and polyvinyl alcohol. The water-insoluble porous carrier may have a coating layer comprising a polymer material having a hydroxyl group, such as a polymer of hydroxyethyl methacrylate, a graft copolymer such as a copolymer of a monomer having a polyethylene oxide chain with another polymerizable monomer, or the like. Among these materials, cellulose or a synthetic polymer such as polyvinyl alcohol is preferably used for practical use because active groups can easily be introduced into the carrier surfaces.

Among these materials, the cellulose carrier is most preferably used. The cellulose carrier has the advantages: (1) It is hardly broken or causes fine particles because of its relatively high mechanical strength and toughness, and thus even if the body fluid is flowed through a column filled with the cellulose carrier at a high flow rate, consolidation little occurs to permit the body fluid to flow at a high speed. (2) It has high safety as compared with a synthetic polymer carrier. Therefore, the cellulose carrier is most preferably used as the water-insoluble porous carrier in the present invention.

As an anticoagulant for an extracorporeal circulation therapy using an adsorber of the present invention, any one of heparin, low-molecular weight heparin, nafamostat mesilate, gabexate mesilate, argatroban, a sodium citrate solution, and a citric acid-containing anticoagulant such as an acid citrate-dextrose solution (ACD solution) and a citrate-phosphate-dextrose solution (CPD solution) may be used. In particular, from the viewpoint of whole blood treatment, a citric acid-containing anticoagulant, heparin, low-molecular weight heparin, or nafamostat mesilate is particularly preferably used as the anticoagulant

The absorbent of the present invention may be used in various methods for adsorbing the low-density lipoproteins and fibrinogen from the body fluid. Representative examples of the methods include a method comprising taking out the body fluid and storing it in a bag or the like, mixing the adsorbent with the body fluid to remove the low-density lipoproteins and fibrinogen, and then filtering off the adsorbent to obtain the body fluid free from the low-density lipoproteins and fibrinogen, and a method comprising preparing an adsorber comprising a container which is filled with the adsorbent and which has a body fluid inlet and outlet, the outlet having a filter for passing the body fluid but not passing the adsorbent, and flowing the body fluid through the adsorber. The absorbent of the present invention may be used in either of the methods, but the latter method comprises a simple operation and can be incorporated into an extracorporeal circulation circuit to permit the on-line efficient removal of the low-density lipoproteins and fibrinogen from the body fluid of a patient. Therefore, the absorbent of the present invention is most preferably used in this method for adsorbing the low-density lipoproteins and fibrinogen using the adsorbent of the present invention.

The adsorber of the present invention comprises a container which is filled with the adsorbent and which has a body fluid inlet and outlet, the outlet having a filter for passing the body fluid but not passing the adsorbent. The capacity of the adsorber of the present invention must be 100 ml or more from the viewpoint of the effect of decreasing the low-density lipoproteins and fibrinogen. Although the capacity of the adsorber is not limited from the viewpoint of adsorption ability, the capacity of the adsorber is preferably 1000 ml or less, and more preferably 800 ml or less, because a blood pressure drop possibly occurs when the amount of the blood taken out from the body is excessively large. The capacity of the adsorber is most preferably 400 ml or less from the viewpoint that even if the adsorber is incorporated into the circuit of another blood purification therapy such as hemodialysis or the like, the amount of the blood extracorporeally circulated is not excessively increased, and a blood pressure drop possibly occurring when blood is taken out from the body can be prevented as much as possible.

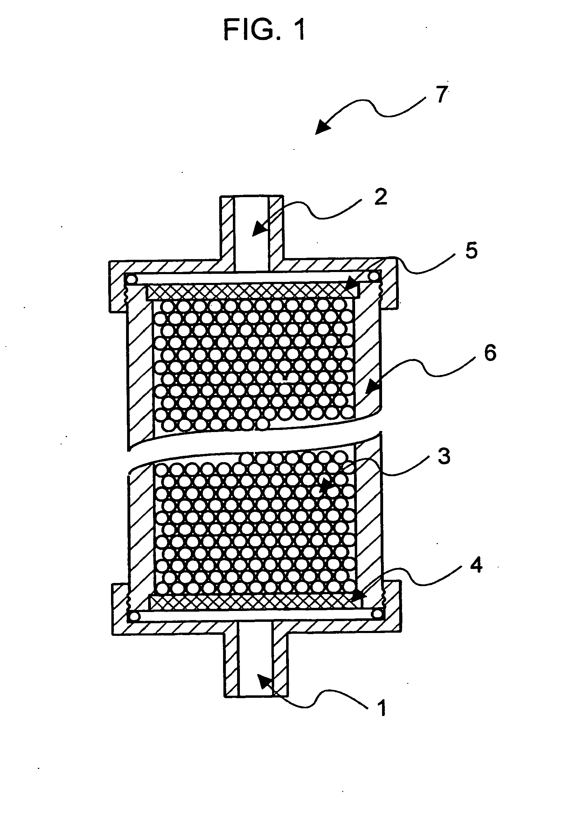

The adsorber of the present invention will be described with reference to Fig. 1 which is a schematic cross-sectional view of an example.

In Fig. 1, reference numeral 1 denotes a fluid inlet, reference numeral 2 denotes a fluid outlet, reference numeral 3 denotes an adsorbent for low-density lipoproteins and fibrinogen, reference numerals 4 and 5 each denote a mesh, reference numeral 6 denotes a column, and reference numeral 7 denotes an adsorber for low-density lipoproteins and fibrinogen. However, the adsorber for low-density lipoproteins and fibrinogen of the present invention is not limited to this example, and the shape of the adsorber is not particularly limited as long as it comprises a container filled with the adsorbent for low-density lipoproteins and fibrinogen, the container having a fluid inlet and outlet and means for preventing an outflow of the adsorbent to the outside.

Best Mode for Carrying Out the Invention

The present invention will be described in detail below with reference to examples.

(REFERENCE EXAMPLE)

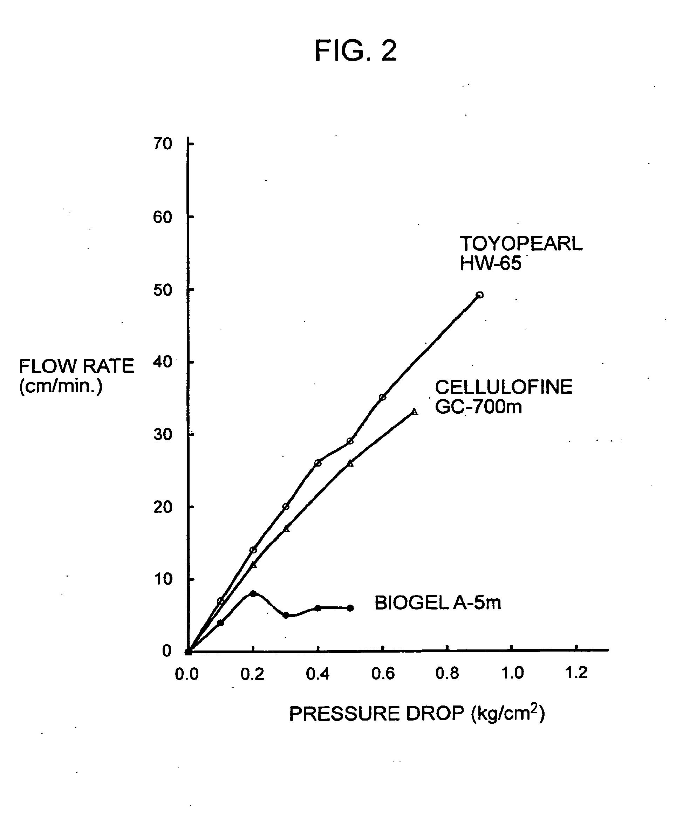

A glass cylindrical column (inner diameter: 9mm, column length: 150 mm) comprising filters (pore size: 15 $\mu$m) provided at both ends was uniformly filled with each of an agarose material (Biogel A-5m produced by Bio-Rad Laboratories, Inc., particle diameter: 50 to 100 mesh), a vinyl polymer material (Toyopearl HW-65 produced by Tosoh Corporation, particle diameter: 50 to 100 $\mu$m), and a cellulose material (Cellulofine GC-700m produced by Chisso Corporation, particle diameter: 45 to 105 $\mu$m). Then, water was flowed through the column by a peristaltic pump to determine the relation between the flow rate and pressure drop $\Delta$P. The results are shown in Fig. 2.

Fig. 2 shows that with Toyopearl HW-65 and Cellulofine GC-700m, the flow rate increases substantially in proportion to increases in pressure, while with BiogelA-5m, the flow rate does not increase due to consolidation even when the pressure is increased. In the present invention, like Toyopearl HW-65 and Cellulofine GC-700m, a material showing a linear relation between the pressure drop $\Delta$P and the flow rate up to 0.3 kgf/cm$^2$ is referred to as a "hard material".

(EXAMPLE 1)

First, 22 ml of water, 31 ml of a 4N NaOH aqueous solution, and 32 ml of epichlorohydrin were added to 100 ml of porous cellulose beads having an average particle diameter of about 450 $\mu$m and a molecular weight exclusion limit of $5 \times 10^7$ for globular proteins, followed by reaction at 40°C for 2 hours under stirring. After the reaction, the beads were sufficiently washed with water to prepare epoxidized cellulose beads. The amount of the epoxy groups of the epoxidized cellulose beads was 16.4 $\mu$mol/ml (wet volume).

On the other hand, 7.5 g of dextran sulfate (sulfur content: about 18%, molecular weight: about 4000) was dissolved in 25 ml of water to prepare an aqueous dextran sulfate solution. Then, 50 ml of the epoxidized cellulose beads wetted with water was added to the aqueous dextran sulfate solution, and the resultant mixture was adjusted to alkali with a NaOH aqueous solution, followed by reaction at 45°C for 1.5 hours. After the reaction, the beads were sufficiently washed with water and brine, and a solution prepared by dissolving 0.77 g of L-tryptophan in 50 ml of a diluted NaOH aqueous solution was added to the beads, followed by reaction at 50°C for 8 hours. Then, the beads were sufficiently washed with water and brine to prepare cellulose beads (A) with immobilized dextran sulfate and tryptophan.

Beads A were charged in an acrylic column (volume 2.7 ml) having an inner diameter of 10 mm and a length of 34 mm and comprising polyethylene terephthalate meshes provided at both ends and each having an opening of 150 $\mu$m. Then, 40 ml of the blood of a healthy adult, which was anticoagulated by adding 5 units of heparin per milliliter of blood, was circulated through the column at a flow rate of 6.5 ml/min for 2 hours. Table 1 shows the numbers of the blood cells in the pooled blood before and after the circulation for 2 hours. All blood cells showed excellent passing property. Table 2 shows the concentrations of LDL-cholesterol, fibrinogen, and HDL-cholesterol in the pooled blood before and after the circulation. As shown in Table 2, LDL-cholesterol is decreased from 116 mg/dl to 78 mg/dl, and fibrinogen is decreased from 132 mg/dl to 93 mg/dl, but HDL-cholesterol is slightly decreased from 66 mg/dl to 62 mg/dl.

The amount of the immobilized tryptophan on beads A was determined from the nitrogen content of the adsorbent. Namely, 1 ml of beads A was sufficiently washed with water, dried under reduced pressure at 60°C for 6 hours or more, and then quantitatively analyzed by a total nitrogen microanalyzer. As a result, the amount of the immobilized tryptophan on beads A was 7.8 $\mu$mol/ml.

The amount of the immobilized dextran sulfate of beads A was measured by utilizing the affinity of dextran sulfate for toluidine blue. Namely, about 100 ml of a toluidine blue (Basic blue 17 (Tokyo Kasei Kogyo Co., Ltd.) aqueous solution adjusted to about 90 mg/l was added to 3 ml of beads A, and the resultant mixture was stirred for 10 minutes and allowed to stand. Then, the amount of the toluidine blue in the supernatant was determined by absorbance at 630 nm, and a decrease in amount of the toluidine blue was determined as the amount of the immobilized dextran sulfate. As a result, the amount of the immobilized dextran on beads A was 0.16 $\mu$mol/ml, and the ratio TR/PA was 48.6.

(EXAMPLE 2)

First, 4 ml of water, 32 ml of a 4N NaOH aqueous solution, and 29 ml of epichlorohydrin were added 100 ml of the same cellulose beads as in EXAMPLE 1, followed by reaction at 40°C for 2 hours under stirring. After the reaction, the beads were sufficiently washed with water to prepare epoxidized cellulose beads. The amount of the epoxy groups of the epoxidized cellulose beads was 19.9 $\mu$mol/ml (wet volume).

On the other hand, 7.5 g of the same dextran sulfate as in EXAMPLE 1 was dissolved in 25 ml of water to prepare an aqueous dextran sulfate solution. Then, 50 ml of the epoxidized cellulose beads wetted with water was added to the aqueous dextran sulfate solution, and the resultant mixture was adjusted to alkali with a NaOH aqueous solution, followed by reaction at 45°C for 3 hours. After the reaction, the beads were sufficiently washed with water and brine, and a solution prepared by dissolving 0.77 g of L-tryptophan in 50 ml of a diluted NaOH aqueous solution was added to the beads, followed by reaction at 55°C for 6 hours. Then, the beads were sufficiently washed with water and brine to prepare cellulose beads (B) with immobilized dextran sulfate and tryptophan. The amount of the immobilized tryptophan on beads B was 7.8 $\mu$mol/ml, the amount of the immobilized dextran sulfate on beads B was 0.23 $\mu$mol/ml, and the TR/PA ratio was 33.8.

Beads B were charged in a column, and 40 ml of the blood of a healthy adult was circulated through the column for 2 hours by the same method as in EXAMPLE 1. Table 1 shows the numbers of the blood cells in the pooled blood before and after the circulation. All blood cells showed excellent passing property. Table 2 shows the concentrations of LDL-cholesterol, fibrinogen, and HDL-cholesterol in the pooled blood before and after the circulation. As shown in Table 2, LDL-cholesterol is decreased from 91 mg/dl to 51 mg/dl, and fibrinogen is decreased from 220 mg/dl to 143 mg/dl, but HDL-cholesterol is slightly decreased from 42 mg/dl to 41 mg/dl.

(EXAMPLE 3)

First, 55 ml of water, 15 ml of a 4N NaOH aqueous solution, and 14 ml of epichlorohydrin were added 100 ml of the same cellulose beads as in EXAMPLE 1, followed by reaction at 40°C for 2 hours under stirring. After the reaction, the beads were sufficiently washed with water to prepare epoxidized cellulose beads. The amount of the epoxy groups of the epoxidized cellulose beads was 8.8 $\mu$mol/ml (wet volume).

On the other hand, 19.8 g of the same dextran sulfate as in EXAMPLE 1 was dissolved in 25 ml of water to prepare an aqueous dextran sulfate solution. Then, 50 ml of the epoxidized cellulose beads wetted with water were added to the aqueous dextran sulfate solution, and the resultant mixture was adjusted to alkali with a NaOH aqueous solution, followed by reaction at 45°C for 6 hours. After the reaction, the beads were sufficiently washed with water and brine, and a solution prepared by dissolving 0.77 g of L-tryptophan in 50 ml of a diluted NaOH aqueous solution was added to the beads, followed by reaction at 50°C for 8 hours. Then, the beads were sufficiently washed with water and brine to prepare cellulose beads (C) with immobilized dextran sulfate and tryptophan. The amount of the immobilized tryptophan on beads C was 4.0 $\mu$mol/ml, the amount of the immobilized dextran sulfate on beads C was 0.32 $\mu$mol/ml, and the TR/PA ratio was 12.5.

Beads C were charged in a column, and 40 ml of the blood of a healthy adult was circulated through the column for 2 hours by the same method as in EXAMPLE 1. Table 1 shows the numbers of the blood cells in the pooled blood before and after the circulation. All blood cells showed excellent passing property. Table 2 shows the concentrations of LDL-cholesterol, fibrinogen, and HDL-cholesterol in the pooled blood before and after the circulation. As shown in Table 2, LDL-cholesterol is decreased from 163 mg/dl to 101 mg/dl, and fibrinogen is decreased from 215 mg/dl to 167 mg/dl, but HDL-cholesterol is slightly decreased from 60 mg/dl to 56 mg/dl.

(COMPARATIVE EXAMPLE 1)

Cellulose beads (D) with immobilized dextran sulfate and tryptophan were prepared by the same method as in EXAMPLE 3 except that the reaction time of dextran sulfate was changed from 6 hours to 0.5 hour, and the amount of dextran sulfate was changed from 19.8 g to 7.5 g. The amount of the immobilized tryptophan on beads D was 5.7 $\mu$mol/ml, the amount of the immobilized dextran sulfate on beads D was 0.08 $\mu$mol/ml, and the TR/PA ratio was 70.9.

Beads D were charged in a column, and 40 ml of the blood of a healthy adult was circulated through the column for 2 hours by the same method as in EXAMPLE 1. Table 1 shows the numbers of the blood cells in the pooled blood before and after the circulation. Although erythrocytes showed excellent passing property, leukocytes and platelets are decreased to 66% and 63%, respectively, after the circulation, and thus showed slightly low passing property. Table 2 shows the concentrations of LDL-cholesterol, fibrinogen, and HDL-cholesterol in the pooled blood before and after the circulation. As shown in Table 2, fibrinogen is decreased from 189 mg/dl to 127 mg/dl, but LDL-cholesterol is slightly decreased from 86 mg/dl to 62 mg/dl, and HDL-cholesterol is slightly decreased from 66 mg/dl to 63 mg/dl.

(COMPARATIVE EXAMPLE 2)

First, 14 ml of water, 24 ml of a 4N NaOH aqueous solution, and 29 ml of epichlorohydrin were added 100 ml of the same cellulose beads as in EXAMPLE 1, followed by reaction at 40°C for 2 hours under stirring. After the reaction, the beads were sufficiently washed with water to prepare epoxidized cellulose beads. The amount of the epoxy groups of the epoxidized cellulose beads was 14.7 $\mu$mol/ml (wet volume).

Then, a solution prepared by dissolving 0.77 g of L-tryptophan in 50 ml of a diluted NaOH aqueous solution was added to 50 ml of the epoxidized cellulose beads, followed by reaction at 55°C for 6 hours. Then, the beads were sufficiently washed with water and brine to prepare cellulose beads (E) with immobilized tryptophan. The amount of the immobilized tryptophan on beads E was 8.2 $\mu$mol/ml.

Beads E were charged in a column, and 40 ml of the blood of a healthy adult was circulated through the column for 2 hours by the same method as in EXAMPLE 1. Table 1 shows the numbers of the blood cells in the pooled blood before and after the circulation. Although erythrocytes and leukocytes showed excellent passing property, platelets are decreased to 69% after the circulation and thus showed slightly low passing property. Table 2 shows the concentrations of LDL-cholesterol, fibrinogen, and HDL-cholesterol in the pooled blood before and after the circulation. As shown in Table 2, fibrinogen is decreased from 132 mg/dl to 77 mg/dl, but LDL-cholesterol is slightly decreased from 116 mg/dl to 85 mg/dl, and HDL-cholesterol is slightly decreased from 66 mg/dl to 61 mg/dl.

(EXAMPLE 4)

First, 42 ml of water, 100 ml of a 2N NaOH aqueous solution, and 17 ml of epichlorohydrin were added 100 ml of porous cellulose beads having an average particle diameter of about 410 $\mu$m and a molecular weight exclusion limit of $5\times10^7$ for globular proteins, followed by reaction at 40°C for 2 hours. After the reaction, the beads were sufficiently washed with water to prepare epoxidized cellulose beads. The amount of the epoxy groups of the epoxidized cellulose beads was 16.5 $\mu$mol/ml (wet volume).

On the other hand, 23.3 g of the same dextran sulfate as in EXAMPLE 1 was dissolved in 39 ml of water to prepare an aqueous dextran sulfate solution. Then, 50 ml of the epoxidized cellulose beads wetted with water was added to the aqueous dextran sulfate solution, and the resultant mixture was adjusted to alkali with a NaOH aqueous solution, followed by reaction at 45°C for 6 hours. After the reaction, the beads were sufficiently washed with water and brine, and a solution prepared by dissolving 0.93 g of L-tryptophan in 50 ml of water by heating was added to the beads. After the resultant mixture was adjusted to alkali with a NaOH aqueous solution, reaction was performed at 50°C for 8 hours. Then, the beads were sufficiently washed with water and brine to prepare cellulose beads (F) with immobilized dextran sulfate and tryptophan. The amount of the immobilized tryptophan on beads F was 7.8 $\mu$mol/ml, the amount of the immobilized dextran sulfate on beads F was 0.17 $\mu$mol/ml, and the TR/PA ratio was 45.9.

Beads F were charged in an acrylic column (volume 3.5 ml) having an inner diameter of 10 mm and a length of 45 mm and comprising polyethylene terephthalate meshes provided at both ends and each having an opening of 50 $\mu$m. Then, 43 ml of the blood of a healthy adult, which was anticoagulated by adding 5 units of heparin per milliliter of blood, was circulated through the column at a flow rate of 2.1 ml/min for 2 hours. Table 3 shows the numbers of the blood cells in the pooled blood before and after the circulation for 2 hours. All blood cells showed excellent passing property. Table 4 shows the concentrations of LDL-cholesterol, fibrinogen, and HDL-cholesterol in the pooled blood before and after the circulation. As shown in Table 4, LDL-cholesterol is decreased from 141 mg/dl to 94 mg/dl, and fibrinogen is decreased from 234 mg/dl to 146 mg/dl, but HDL-cholesterol is slightly decreased from 49 mg/dl to 45 mg/dl.

(COMPARATIVE EXAMPLE 3)

First, 42 ml of water, 50 ml of a 2N NaOH aqueous solution, and 17 ml of epichlorohydrin were added 100 ml of the same cellulose beads as in EXAMPLE 4, followed by reaction at 40°C for 2 hours under stirring. After the reaction, the beads were sufficiently washed with water to prepare epoxidized cellulose beads. The amount of the epoxy groups of the epoxidized cellulose beads was 12.4 $\mu$mol/ml (wet volume).

Then, 23.3 g of the same dextran sulfate in EXAMPLE 1 was dissolved in 39 ml of water to prepare an aqueous dextran sulfate solution, and 50 ml of the epoxidized cellulose beads wetted with water was added to the aqueous dextran sulfate solution. After the resultant mixture was adjusted to alkali with a NaOH aqueous solution, reaction was performed at 45°C for 20 hours. Then, the beads were sufficiently washed with water and brine to prepare cellulose beads (G) with immobilized dextran sulfate. The amount of the immobilized dextran sulfate on beads G was 0.6 $\mu$mol/ml.

Beads G were charged in a column, and 43 ml of the blood of a healthy adult was circulated through the column for 2 hours by the same method as in EXAMPLE 4. Table 3 shows the numbers of the blood cells in the pooled blood before and after the circulation. Although erythrocytes showed excellent passing property, leukocytes and platelets are decreased to 66% and 63%, respectively, after the circulation and thus showed slightly low passing property. Table 4 shows the concentrations of LDL-cholesterol, fibrinogen, and HDL-cholesterol in the pooled blood before and after the circulation. As shown in Table 4, fibrinogen is decreased from 141 mg/dl to 89 mg/dl, but LDL-cholesterol is slightly decreased from 234 mg/dl to 198 mg/dl, and HDL-cholesterol is slightly decreased from 49 mg/dl to 46 mg/dl.

(EXAMPLE 5)

First, 1.0 ml of cellulose beads (B) with immobilized dextran sulfate and tryptophan prepared in EXAMPLE 2 was measured, and 10 ml of the plasma of a healthy person was added to the beads, followed by incubation at 37°C for 4 hours. After incubation, plasma was separated from the beads, and the concentrations of LDL-cholesterol, fibrinogen, albumin, IgG, and HDL-cholesterol of the plasma were measured. The results are shown in Table 5. As shown in Table 5, LDL-cholesterol is decreased from 115 mg/dl to 81 mg/dl, and fibrinogen is decreased from 244 mg/dl to 186 mg/dl, but albumin is slightly decreased from 4.5 g/dl to 4.3 g/dl, IgG is slightly decreased from 1203 mg/dl to 1133 mg/dl, and HDL-cholesterol is slightly decreased from 62 mg/dl to 59 mg/dl.

(EXAMPLE 6)

First, 1.0 ml of cellulose beads (F) with immobilized dextran sulfate and tryptophan prepared in EXAMPLE 4 was measured, and 10 ml of the plasma of a healthy adult was added to the beads, followed by incubation at 37°C for 4 hours. After incubation, plasma was separated from the beads, and the concentrations of LDL-cholesterol, fibrinogen, albumin, IgG, and HDL-cholesterol of the plasma were measured. The results are shown in Table 5. As shown in Table 5, LDL-cholesterol is decreased from 87 mg/dl to 62 mg/dl, and fibrinogen is decreased from 260 mg/dl to 190 mg/dl, but albumin is slightly decreased from 4.7 g/dl to 4.5 g/dl, IgG is slightly decreased from 927 mg/dl to 876 mg/dl, and HDL-cholesterol is slightly decreased from 55 mg/dl to 54 mg/dl.

Table 1

| | Adsorbent | Average particle diameter [$\mu$m] | Amount of immobilized dextran sulfate | Amount of immobilized tryptophan | TR/PA ratio | Number of leukocytes | | | Number of platelets | | | Number of erythrocytes | | |
|---|---|---|---|---|---|---|---|---|---|---|---|---|---|---|
| | | | [$\mu$mol/ml] | [$\mu$mol/ml] | [-] | [$\times 10^2/\mu$l] | | Ratio* [%] | [$\times 10^4/\mu$l] | | Ratio* [%] | [$\times 10^4/\mu$l] | | Ratio* [%] |
| | | | | | | Before blood perfusion | Afterblood perfusion | | Before blood perfusion | Afterblood perfusion | | Before blood perfusion | Afterblood perfusion | |
| Example 1 | A | 450 | 0.16 | 7.8 | 48.6 | 40 | 34 | 85 | 18.1 | 13.6 | 75 | 493 | 499 | 101 |
| Example 2 | B | 450 | 0.23 | 7.8 | 33.8 | 55 | 48 | 87 | 16.8 | 12.4 | 74 | 504 | 510 | 101 |
| Example 3 | C | 450 | 0.32 | 4.0 | 12.5 | 59 | 52 | 88 | 22.7 | 17.9 | 79 | 498 | 500 | 100 |
| Comp. Example 1 | D | 450 | 0.08 | 5.7 | 70.9 | 47 | 31 | 66 | 19.0 | 12.0 | 63 | 441 | 444 | 101 |
| Comp. Example 2 | E | 450 | 0 | 8.2 | - | 40 | 35 | 88 | 18.1 | 12.4 | 69 | 493 | 503 | 102 |
| Ratio*: After blood perfusion/before blood perfusion $\times$ 100 | | | | | | | | | | | | | | |

Table 2

| | Adsorbent | Average particle diameter [μm] | Amount of immobiliz-ed dextran sulfate [μmol/ml] | Amount of immobiliz-ed tryptophan [μmol/ml] | TR/PA ratio [-] | LDL-cholesterol | | | Fibrinogen | | | HDL-cholesterol | | |
|---|---|---|---|---|---|---|---|---|---|---|---|---|---|---|
| | | | | | | [mg/dl] | | Rate of Decrease [%] | [mg/dl] | | Rate of Decrease [%] | [mg/dl] | | Rate of decrease [%] |
| | | | | | | Before *) | After *) | | Before *) | After *) | | Before *) | After *) | |
| Example 1 | A | 450 | 0.16 | 7.8 | 48.6 | 116 | 78 | 33 | 132 | 93 | 30 | 66 | 62 | 6 |
| Example 2 | B | 450 | 0.23 | 7.8 | 33.8 | 91 | 51 | 44 | 220 | 143 | 35 | 42 | 41 | 2 |
| Example 3 | C | 450 | 0.32 | 4.0 | 12.5 | 163 | 101 | 38 | 215 | 167 | 22 | 60 | 56 | 7 |
| Comp. Example 1 | D | 450 | 0.08 | 5.7 | 70.9 | 86 | 62 | 28 | 189 | 127 | 33 | 66 | 63 | 5 |
| Comp. Example 2 | E | 450 | 0 | 8.2 | - | 116 | 85 | 27 | 132 | 77 | 42 | 66 | 61 | 8 |

Before: Before blood perfusion    After: After blood perfusion

Table 2 (continued)

| | Amount of adsorption | | |
|---|---|---|---|
| | LDL-cholesterol [mg/mL-gel] | Fibrinogen [mg/mL-gel] | HDL-cholesterol [mg/mL-gel] |
| Example 1 | 3.1 | 3.2 | 0.2 |
| Example 2 | 3.2 | 6.2 | 0.1 |
| Example 3 | 5.0 | 3.9 | 0.3 |
| Comp. Example 1 | 2.0 | 5.3 | 0.3 |
| Comp. Example 2 | 2.6 | 4.6 | 0.4 |

Table 3

| | Adsorbent | Average particle di- ameter [μm] | Amount of immobilized dextran sulfate | Amount of immobilized tryptophan | TR/PA ratio | Number of leukocytes | | | Number of platelets | | | Number of erythrocytes | | |
|---|---|---|---|---|---|---|---|---|---|---|---|---|---|---|
| | | | [μmol/ml] | [μmol/ml] | [-] | [×10²/μl] | | Ratio* [%] | [×10⁴/μl] | | Ratio* [%] | [×10⁴/μl] | | Ratio* [%] |
| | | | | | | Before blood per- fusion | Afterblood perfusion | | Before blood per- fusion | Afterblood perfusion | | Before blood per- fusion | Afterblood perfusion | |
| Example 4 | F | 410 | 0.17 | 7.8 | 45.9 | 59 | 52 | 88 | 22.7 | 17.9 | 79 | 498 | 500 | 100 |
| Comp. Example 3 | G | 410 | 0.6 | 0 | 0 | 47 | 31 | 66 | 19.0 | 12.0 | 63 | 441 | 444 | 101 |
| Ratio*: After blood perfusion/before blood perfusion × 100 | | | | | | | | | | | | | | |

## Table 4

| | Adsorbent | Average particle diameter [μm] | Amount of immobiliz-ed dextran sulfate [μmol/ml] | Amount of immobiliz-ed tryptophan [μmol/ml] | TR/PA ratio [-] | LDL-cholesterol [mg/dl] Before *) | After *) | Rate of Decrease [%] | Fibrinogen [mg/dl] Before *) | After *) | Rate of Decrease [%] | HDL-cholesterol [mg/dl] Before *) | After *) | Rate of decrease [%] |
|---|---|---|---|---|---|---|---|---|---|---|---|---|---|---|
| Example 4 | F | 410 | 0.17 | 7.8 | 45.9 | 141 | 94 | 33 | 234 | 146 | 38 | 49 | 45 | 8 |
| Comp. Example 3 | G | 410 | 0.6 | 0 | 0 | 141 | 89 | 37 | 234 | 198 | 15 | 49 | 46 | 6 |

Before: Before blood perfusion   After: After blood perfusion

## Table 4 (continued)

| | Amount of adsorption LDL-cholesterol [mg/mL-gel] | Fibrinogen [mg/mL-gel] | HDL-cholesterol [mg/mL-gel] |
|---|---|---|---|
| Example 4 | 3.2 | 5.9 | 0.3 |
| Comp. Example 3 | 3.5 | 2.4 | 0.2 |

## Table 5

| Measurement items | Absorbent | Average particle diameter [μm] | LDL-cholesterol [mg/dl] Before adsorption | After adsorption | Rate of Decrease [%] | Fibrinogen [mg/dl] Before adsorption | After adsorption | Rate of Decrease [%] | Albumin [g/dl] Before adsorption | After adsorption | Rate of Decrease [%] |
|---|---|---|---|---|---|---|---|---|---|---|---|
| Example 5 | B | 450 | 115 | 81 | 30 | 244 | 186 | 24 | 4.5 | 4.3 | 4 |
| Example 6 | F | 410 | 87 | 62 | 29 | 260 | 190 | 27 | 4.7 | 4.5 | 4 |

## Table 5 (continued)

| Measurement items | IgG [mg/dl] Before adsorption | After adsorption | Rate of Decrease [%] | HDL-cholesterol [mg/dl] Before adsorption | After adsorption | Rate of Decrease [%] |
|---|---|---|---|---|---|---|
| Example 5 | 1203 | 1133 | 6 | 62 | 59 | 5 |
| Example 6 | 927 | 876 | 6 | 55 | 54 | 2 |

Brief Description of the Drawings

Fig. 1 is a schematic cross-sectional view showing an example of an adsorber of the present invention.

Reference numerals each denote the following:

1 body fluid inlet
2 body fluid outlet
3 adsorbent for low-density lipoproteins and fibrinogen
4, 5 mesh (means for preventing adsorbent outflow)
6 column
7 adsorber for low-density lipoproteins and fibrinogen

Fig. 2 is a graph showing the relation between the flow rate and pressure drop in the use of three types of gels.

Industrial Applicability

According to the present invention, low-density lipoproteins and fibrinogen can be efficiently adsorbed directly from a body fluid, particularly whole blood, to decrease the concentrations of these components in the body fluid with minimizing losses of useful substances such as HDL and albumin. The present invention is particularly effective as a method for decreasing the concentrations of low-density lipoproteins and fibrinogen in the blood of a patient with arteriosclerosis, particularly arteriosclerosis obliterans.

**Claims**

1. An adsorbent capable of whole blood treatment for adsorbing low-density lipoproteins and fibrinogen, the adsorbent comprising a polyanionic compound which is immobilized on a water-insoluble porous carrier **characterized in that** the adsorbent further comprises a tryptophan derivative which is immobilized on the water-insoluble porous carrier, wherein the amount of the immobilized polyanionic compound is 0.10 $\mu$mol to 1.5 $\mu$mol per milliliter of wet volume of the adsorbent, and the molar ratio of the amount of the immobilized tryptophan derivative to the amount of the immobilized polyanionic compound is 1 to 70.

2. The adsorbent capable of whole blood treatment for adsorbing low-density lipoproteins and fibrinogen according to claim 1, wherein the polyanionic compound is dextran sulfate.

3. The adsorbent capable of whole blood treatment for adsorbing low-density lipoproteins and fibrinogen according to claim 1 or 2, wherein the tryptophan derivative is tryptophan.

4. The adsorbent capable of whole blood treatment for adsorbing low-density lipoproteins and fibrinogen according to any one of claims 1 to 3, wherein the water-insoluble porous carrier is a cellulose carrier.

5. The adsorbent capable of whole blood treatment for adsorbing low-density lipoproteins and fibrinogen according to any one of claims 1 to 4, wherein the water-insoluble porous carrier has a molecular weight exclusion limit of $5 \times 10^5$ to $1 \times 10^8$ for globular proteins.

6. An adsorber capable of whole blood treatment for absorbing low-density lipoproteins and fibrinogen, the adsorber comprising a container having a fluid inlet, a fluid outlet, and means for preventing an outflow of an adsorbent to the outside, wherein the container is filled with the adsorbent capable of whole blood treatment for adsorbing low-density lipoproteins and fibrinogen according to any one of claims 1 to 5.

7. The adsorber capable of whole blood treatment for absorbing low-density lipoproteins and fibrinogen according to claim 6, wherein the capacity of the adsorber is 100 ml to 400 ml.

**Patentansprüche**

1. Adsorptionsmittel, geeignet zur Vollblutbehandlung zum Adsorbieren von Low Density Lipoproteinen und Fibrinogen, wobei das Adsorptionsmittel eine polyanionische Verbindung, die auf einem wasserunlöslichen porösen Träger immobilisiert ist, umfasst, **dadurch gekennzeichnet, dass** das Adsorptionsmittel zusätzlich ein Tryptophan-Derivat umfasst, das auf dem wasserunlöslichen porösen Träger immobilisiert ist, wobei die Menge der immobilisierten polyanionischen Verbindung 0,10 $\mu$mol bis 1,5 $\mu$mol pro Milliliter nasses Volumen des Adsorptionsmittels beträgt und das Molverhältnis der Menge des immobilisierten Tryptophan-Derivats zu der Menge der immobilisierten polyanionischen Verbindung 1 bis 70 ist.

2. Adsorptionsmittel, geeignet zur Vollblutbehandlung zum Adsorbieren von Low Density Lipoproteinen und Fibrinogen, nach Anspruch 1, in dem die polyanionische Verbindung Dextransulfat ist.

3. Adsorptionsmittel, geeignet zur Vollblutbehandlung zum Adsorbieren von Low Density Lipoproteinen und Fibrinogen, nach Anspruch 1 oder 2, in dem das Tryptophan-Derivat Tryptophan ist.

4. Adsorptionsmittel, geeignet zur Vollblutbehandlung zum Adsorbieren von Low Density Lipoproteinen und Fibrinogen, nach einem der Ansprüche 1 bis 3, in dem der wasserunlösliche poröse Träger ein Celluloseträger ist.

5. Adsorptionsmittel, geeignet zur Vollblutbehandlung zum Adsorbieren von Low Density Lipoproteinen und Fibrinogen, nach einem der Ansprüche 1 bis 4, in dem der wasserunlösliche poröse Träger eine Molekulargewichtsausschlussgrenze für globuläre Proteine von $5 \times 10^5$ bis $1 \times 10^8$ aufweist.

6. Adsorber, geeignet zur Vollblutbehandlung zum Adsorbieren von Low Density Lipoproteinen und Fibrinogen, wobei der Adsorber einen Behälter mit einem Flüssigkeitseinlass, einem Flüssigkeitsauslass und Mittel zum Verhindern eines Ausfließens eines Adsorptionsmittels nach außen umfasst, wobei der Behälter mit dem Adsorptionsmittel, geeignet zur Vollblutbehandlung zum Adsorbieren von Low Density Lipoproteinen und Fibrinogen, nach einem der Ansprüche 1 bis 5 gefüllt ist.

**7.** Adsorber, geeignet zur Vollblutbehandlung zum Adsorbieren von Low Density Lipoproteinen und Fibrinogen, nach Anspruch 6, wobei die Kapazität des Adsorbers 100 ml bis 400 ml beträgt.

**Revendications**

**1.** Adsorbant capable d'un traitement de sang total pour l'adsorption de lipoprotéines de basse densité et de fibrinogène, l'adsorbant comprenant un composé polyanionique qui est immobilisé sur un support poreux insoluble dans l'eau **caractérisé en ce que** l'adsorbant comprend en outre un dérivé de tryptophane qui est immobilisé sur le support poreux insoluble dans l'eau, dans lequel la quantité du composé polyanionique immobilisé est comprise entre 0,10 $\mu$mol et 1,5 $\mu$mol par millilitre de volume humide de l'adsorbant, et le rapport molaire de la quantité du dérivé de tryptophane immobilisé sur la quantité du composé polyanionique immobilisé est compris entre 1 et 70.

**2.** Adsorbant capable d'un traitement de sang total pour l'adsorption de lipoprotéines de basse densité et de fibrinogène selon la revendication 1, dans lequel le composé polyanionique est du sulfate de dextrane.

**3.** Adsorbant capable d'un traitement de sang total pour l'adsorption de lipoprotéines de basse densité et de fibrinogène selon la revendication 1 ou 2, dans lequel le dérivé de tryptophane est du tryptophane.

**4.** Adsorbant capable d'un traitement de sang total pour l'adsorption de lipoprotéines de basse densité et de fibrinogène selon l'une quelconque des revendications 1 à 3, dans lequel le support poreux insoluble dans l'eau est un support de cellulose.

**5.** Adsorbant capable d'un traitement de sang total pour l'adsorption de lipoprotéines de basse densité et de fibrinogène selon l'une quelconque des revendications 1 à 4, dans lequel le support poreux insoluble dans l'eau a une limite d'exclusion de poids moléculaire comprise entre $5 \times 10^5$ et $1 \times 10^8$ pour des protéines globulaires.

**6.** Adsorbeur capable d'un traitement de sang total pour l'adsorption de lipoprotéines de basse densité et de fibrinogène, l'adsorbeur comprenant un récipient ayant une entrée de fluide, une sortie de fluide, et un moyen pour empêcher un écoulement d'un adsorbant à l'extérieur, dans lequel le récipient est rempli d'adsorbant permettant un traitement de sang total pour l'adsorption de lipoprotéines de basse densité et de fibrinogène selon l'une quelconque des revendications 1 à 5.

**7.** Adsorbeur capable d'un traitement de sang total pour l'adsorption de lipoprotéines de basse densité et de fibrinogène selon la revendication 6, dans lequel la capacité de l'adsorbeur est comprise entre 100 ml et 400 ml.

## FIG. 1

## FIG. 2

FLOW RATE (cm/min.) vs PRESSURE DROP (kg/cm²)

TOYOPEARL HW-65

CELLULOFINE GC-700m

BIOGEL A-5m

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7136256 A
- EP 0225867 A
- US 20020164644 A

### Non-patent literature cited in the description

- **W. B. KANNEL et al.** *The Journal of the American Medical Association,* 1987, vol. 258, 1183-1186
- **P. POREDOS et al.** *Angiology,* 1996, vol. 47 (3), 253-259
- **YOSHIE KONNO et al.** *Japanese Journal of Apheresis,* 2003, vol. 22 (1), 44-50
- Amino Acid Fermentation. Kyoritsu Shuppan, 1972, 43-45